# EUROPÄISCHE PATENTANMELDUNG

(11) **EP 1 654 985 A1**
(43) Veröffentlichungstag der Anmeldung: **10.05.2006**
(21) Anmeldenummer: 04026545.6
(22) Anmeldetag: 09.11.2004
(51) Int. Cl.: A61B 5/15, A61B 5/00

(54) **Probenentnahmesystem für Probenflüssigkeit**

(71) Anmelder: F. HOFFMANN-LA ROCHE AG, 4070 Basel (CH); Roche Diagnostics GmbH, 68305 Mannheim (DE)
(72) Erfinder: Calasso, Dr.Irio, 6415 Arth (CH); Sarofim, Emad, 6300 Zug (CH)
(74) Vertreter: Pfiz, Thomas

(57) **Zusammenfassung**

Die Erfindung betrifft ein Probenentnahmesystem für Probenflüssigkeit mit einem Träger (12), einem daran angeordneten Stechelement (14) und einem halboffenen Kanal (16) zum Kapillartransport der Probenflüssigkeit von dem Stechelement (14) zu einer Sammelstelle (18) auf dem Träger (12). Erfindungsgemäß ist eine Aufnahmestruktur (20) für seitlich aus dem Kanal (16) austretende überschüssige Probenflüssigkeit vorgesehen.

## Beschreibung

Die Erfindung betrifft ein Probenentnahmesystem für Probenflüssigkeit, insbesondere Mikroprobenehmer für Blut, mit einem Träger, einem daran angeordneten Stechelement und einem vorzugsweise halboffenen Kanal zum Kapillartransport der Probenflüssigkeit von dem Stechelement zu einer Sammelstelle auf dem Träger.

Die DE-A-101 34 650 offenbart eine disposible Stecheinheit zur Entnahme kleiner Körperflüssigkeitsmengen, die einen Haltebereich aufweist, mit dem das proximale Ende einer länglichen Kapillarstruktur mit mindestens einem Kapillarkanal zum Transport von Körperflüssigkeit verbunden ist und das distale Ende der Kapillarstruktur geeignet ist, Haut zu durchstechen, wobei der mindestens eine Kapillarkanal zumindest über einen Teil seiner Länge nach außen offen ist. Durch die offene Kapillarstruktur ergeben sich neben Vorteilen in der Herstellung auch Verbesserungen hinsichtlich der Probenaufnahme bei kleinen Einstichwunden, wie sie zur Reduzierung des Einstichschmerzes angestrebt werden. Durch den kapillargetriebenen Blutfluss zur Nachweiszone bzw. Sammelstelle kann die Probenentnahme, der Transport und der Nachweis beispielsweise von Blutzucker in einem System integriert werden. Zur Verbesserung der Messung wurde bereits vorgeschlagen, flächig ausgedehnte Nachweisbereiche über die Haupttransportkapillare zu beaufschlagen. Auch hierbei besteht jedoch das Problem, dass bei einem Überschuss des Blutflusses an der Entnahmestelle im Vergleich zum Transportvermögen des Kanals ein ungewollter Blutaustritt auf der Transportstrecke zu einer Kontamination des Systems mit potentiell gefährlicher Bioflüssigkeit führen kann. Zudem wird durch einen solchen ungezielten Blutfluss die Funktionalität des Entnahmesystems verschlechtert.

Ausgehend hiervon liegt der Erfindung die Aufgabe zugrunde, die im Stand der Technik aufgetretenen Nachteile zu überwinden und eine Reduzierung der Kontaminationsgefahr zu erreichen.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen eventuellen Flüssigkeitsüberschuss auf der Transportstrecke gezielt abzuzweigen. Dementsprechend wird erfindungsgemäß eine Aufnahmestruktur für seitlich aus dem Kanal austretende überschüssige Probenflüssigkeit vorgeschlagen. Damit wird erreicht, dass der Überschuss funktionell auf dem Träger festgehalten wird, so dass keine undefinierte Verschmutzung auftritt. Dies ist vor allem wichtig, wenn die Kapillarität des Kanals oder die Flussmenge darin gering ist, wie es bei sehr kleinen Kapillaren oder Kapillarem mit kleinem Aspektverhältnis der Fall ist. Erfindungsgemäß wird gleichsam eine Ventilfunktion geschaffen, die bei Erreichen einer kritischen Fließmenge automatisch wirksam wird. Dadurch ergeben sich besondere Vorteile für die Handhabung solcher Systeme als Wegwerfartikel in tragbaren Instrumenten insbesondere für die routinemäßige Blutzuckerbestimmung bei Diabetes.

Vorteilhafterweise ist die Aufnahmestruktur zumindest in einem dem Kanal nahen Eintrittsbereich für die Probenflüssigkeit als Kapillare wirksam, so dass ein selbsttätiger Ansaugeffekt erzielt wird.

In besonders bevorzugter Ausführung ist die kapillare Transportfähigkeit für die Probenflüssigkeit in Richtung des Kanals größer als in Abzweigrichtung der Aufnahmestruktur. Durch die eingestellten Kapillaritäts-Unterschiede wird die Flüssigkeit nicht daran gehindert, weiterhin in Richtung der Sammelstelle zu fließen.

Vorteilhafterweise ist die Aufnahmestruktur an einem in Fließrichtung gesehen dem Stechelement nachgeordneten und der Sammelstelle vorgeordneten Mittelabschnitt des Kanals auf dem Träger angeordnet. Wenn das Stechelement als Spitze an einer Schaftpartie des Trägers absteht, ist es von Vorteil, wenn die Aufnahmestruktur im Bereich der Schaftpartie angeordnet ist.

Gemäß einer vorteilhaften Ausgestaltung ist die Aufnahmestruktur durch ein unter Freihaltung eines Kapillarspalts gegenüber dem Kanal an dem Träger angeordnetes Deckelelement gebildet. Dadurch wird die in den Kapillarspalt gezogene Flüssigkeitsmenge zugleich nach außen abgeschirmt. Dabei sollte gewährleistet sein, dass der Kapillarspalt eine geringere Kapillarattraktion für die Probenflüssigkeit als der daran angrenzende Kanalabschnitt aufweist, so dass ein Nachfließen in dem Kanal möglich ist.

Eine weitere vorteilhafte Ausgestaltung, welche auch die Herstellung vereinfacht, sieht vor, dass die Aufnahmestruktur durch mindestens einen seitlich neben dem Kanal auf dem Trägerteil angeordnete, vorzugsweise halboffene Überlaufkapillare gebildet ist. Günstig ist es, wenn zwei Überlaufkapillaren beidseitig von dem Kanal vorzugsweise symmetrisch zueinander angeordnet sind. Um das Fassungsvermögen zu erhöhen, kann die mindestens eine Überlaufkapillare mindestens eine Verzweigung aufweisen.

Um den regulären Probentransport nicht unnötig zu beeinflussen, ist es von Vorteil, wenn der Kanal durch eine Seitenwandung von der mindestens einen Überlaufkapillare getrennt ist, wobei überschüssige Probenflüssigkeit über die Seitenwandung in die Überlaufkapillare überfließt. Alternativ ist es auch möglich, dass der Kanal über eine kapillare Abzweigung mit der mindestens einen Überlaufkapillare fluidisch verbunden ist. Dabei sollte sichergestellt sein, dass die Abzweigung einen kleineren Fließquerschnitt als die anschließende Überlaufkapillare aufweist.

Eine weitere Verbesserung wird dadurch erzielt, dass die Aufnahmestruktur zugleich ein Reservoir für die Rückfüllung des Kanals bildet.

Herstellungstechnisch ist es von Vorteil, wenn das Stechelement an einem Flachformteil als Träger angeformt ist, und wenn der Kanal rillenförmig linear ausgebildet ist. In bevorzugter Ausgestaltung sind der Träger und das Stechelement durch photochemisches Maskenätzen einstückig aus einem Flachmaterial gebildet.

Ein weiterer Erfindungsaspekt umfasst ein Blutanalysegerät mit mindestens einem vorzugsweise als Einwegprodukt ausgebildeten erfindungsgemäßen Probenentnahmesystem.

Im Folgenden wird die Erfindung anhand der in der Zeichnung schematisch dargestellten Ausführungsbeispiele näher erläutert. Es zeigen
- Fig. 1: einen Mikroprobenehmer für Blut in der Draufsicht;
- Fig. 2 a und b: einen Schnitt entlang der Linie 2 - 2 der Fig. 1 bei verschiedenem Blutfluss;
- Fig. 3 und 4: weitere Ausführungsbeispiele in der Draufsicht.

Die in der Zeichnung dargestellten Mikroprobenehmer 10 (microsampler) bestehen im Wesentlichen aus einem Träger 12, einem daran angeformten Stechelement 14, einem halboffenen Kanal 16 zum Bluttransport von dem Stechelement 14 zu einer Sammelstelle 18 und einer Aufnahmestruktur 20 für eventuell seitlich aus dem Kanal 16 austretendes überschüssiges Blut.

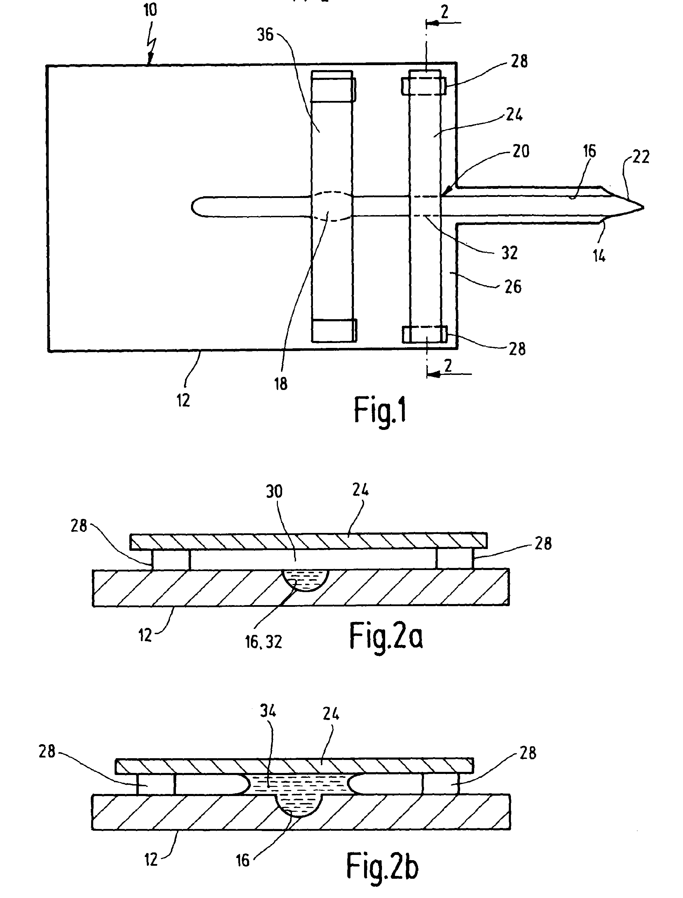

Der in Fig. 1 gezeigte Träger 12 ist mit dem daran ausgebildeten Stechelement 14 in an sich bekannter Weise durch fotochemisches Maskenätzen aus einem dünnen Edelstahlblech gebildet. Die Nadelspitze 22 des Stechelements 14 lässt sich zur Gewinnung einer mikroskopischen Menge (Nano- bis Mikroliter) beispielsweise in die Fingerbeere eines Probanden einstechen, wobei das Blut durch die Kapillarität des Kanals 16 selbsttätig zu der Sammelstelle 18 transportiert wird. Um eine ungewollte Kontamination zu vermeiden, ist die Aufnahmestruktur 20 zur definierten Aufnahme von überschüssigem Blut ausgebildet.

Zu diesem Zweck ist bei dem Ausführungsbeispiel gemäß Fig. 1 und 2 ein Deckelelement 24 als Aufnahmestruktur 20 vorgesehen. Das Deckelelement 24 überspannt den Kanal 16 in einem an das Stechelement 14 angrenzenden Schaftbereich 26 des Trägers 12. Es ist durch Abstandshalter 28 in geringem Abstand zu dem Träger 12 gehalten, so dass ein Kapillarspalt 30 über dem darunter liegenden Kanalabschnitt 32 frei bleibt. Der Kapillarspalt 30 weist eine geringere Kapillarattraktion für Blut als der Kanalabschnitt 32 auf. Auf diese Weise wird das im Kanal 16 fließende Blut nicht daran gehindert, die Sammelstelle 18 zu erreichen. Nur wenn die aus der Einstichstelle abfließende Blutmenge größer als die Fassungskapazität des Kanals 16 ist, wird der Blutüberschuss 34 örtlich definiert in dem Kapillarspalt 30 unter dem Deckelement 24 festgehalten. Gegebenenfalls kann der Überschuss 34 auch für eine Rückfüllung des Kanals 16 bei nachträglich abnehmender Fließmenge aus der Einstichstelle dienen.

Der über seine Länge halboffene rillenförmige Kanal 16 verläuft linear von der Nadelspitze 22 bis über die Sammelstelle 18 hinaus. An der seitlich verbreiterten Sammel- bzw. Zielstelle 18 steht ein Nachweiselement 36 in fluidischem Kontakt mit dem dort angesammelten Blut. Das Nachweiselement 36 spricht auf einen Analyten, beispielsweise Glucose im Blut an, so dass durch eine nicht gezeigte Detektionseinheit ein quantitativer Nachweis geführt werden kann.

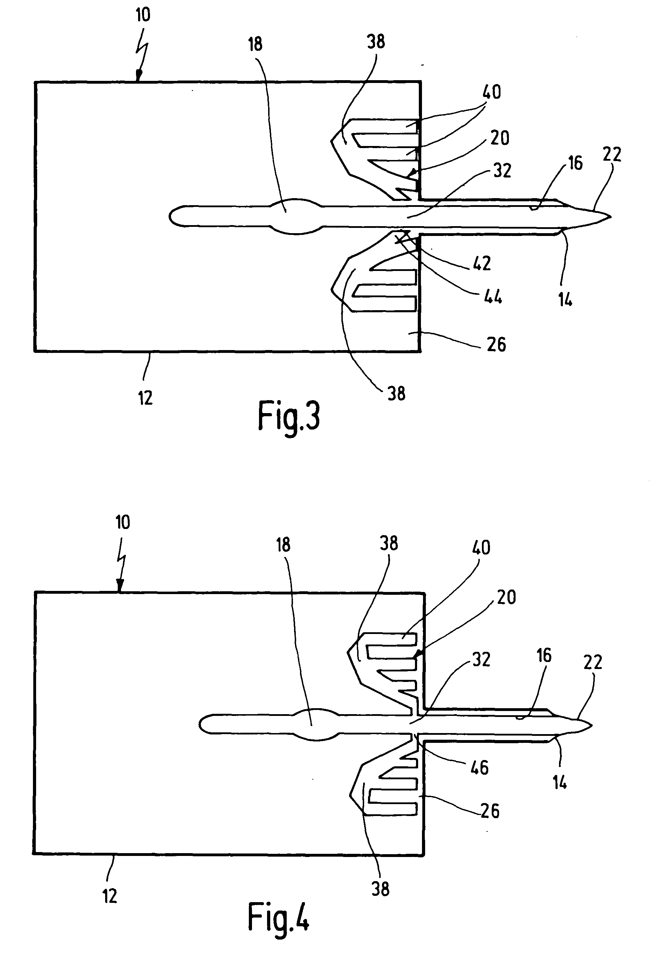

Bei den in Fig. 3 und 4 gezeigten Ausführungsbeispielen sind anstelle eines Deckelelements Überlaufkapillaren 38 als Aufnahmestruktur 20 für überschüssiges Blut vorgesehen. Die Überlaufkapillaren 38 sind jeweils im Schaftbereich 26 des Trägerteils 12 paarweise auf beiden Seiten des Kanals 16 symmetrisch zueinander angeordnet. Um die Aufnahmekapazität zu erhöhen, sind Verzweigungen 40 vorgesehen.

In der Ausführung nach Fig. 3 sind die Überlaufkapillaren 38 durch eine Seitenwandung 42 von dem angrenzenden Kanalabschnitt 32 getrennt, so dass dessen Kapillarität nicht geschwächt wird. Auch hier tritt nur bei einem übermäßigen Zufluss überschüssiges Blut über die Seitenwandung 42 in die Überlaufkapillaren 38. Der Überschuss wird dabei durch die Kapillaraktivität zumindest des kanalseitigen Eintrittsbereichs 44 der Überlaufkapillaren 38 selbsttätig aufgenommen.

Bei der in Fig. 4 gezeigten Ausführungsform sind die Überlaufkapillaren 38 über jeweils eine Abzweigung 46 direkt mit dem Kanal 16 verbunden. Dabei ist es vorteilhaft, wenn die Abzweigung 46 eine sehr kurze und dünne Verbindungskapillare bildet. Auch hier sollte sichergestellt sein, dass die kapillare Transportfähigkeit in Richtung des Kanals 16 größer als in Abzweigrichtung ist.

Die Mikroprobenehmer 10 lassen sich als so genannte Disposables bzw. Einwegprodukte in tragbaren Blutzuckermessgeräten patientennah einsetzen, um so mit geringem Handhabungsaufwand und Einstichschmerz hygienisch einwandfrei Blutzuckerbestimmungen in täglicher Routine durchführen zu können.

## Patentansprüche

1. Probenentnahmesystem für Probenflüssigkeit, insbesondere Mikroprobenehmer für Blut, mit einem Träger (12), einem daran angeordneten Stechelement (14) und einem vorzugsweise halboffenen Kanal (16) zum Kapillartransport der Probenflüssigkeit von dem Stechelement (14) zu einer Sammelstelle (18) auf dem Träger (12), **gekennzeichnet durch** eine Aufnahmestruktur (20) für seitlich aus dem Kanal (16) austretende überschüssige Probenflüssigkeit.

2. Probenentnahmesystem nach Anspruch 1, **dadurch gekennzeichnet, dass** die Aufnahmestruktur (20) zumindest in einem kanalseitigen Eintrittsbereich (44) für die Probenflüssigkeit als Kapillare wirksam ist.

3. Probenentnahmesystem nach Anspruch 1 oder 2, **dadurch gekennzeichnet, dass** die kapillare Transportfähigkeit für die Probenflüssigkeit in Richtung des Kanals (16) größer als in Abzweigrichtung der Aufnahmestruktur (20) ist.

4. Probenentnahmesystem nach einem der Ansprüche 1 bis 3, **dadurch gekennzeichnet, dass** die Aufnahmestruktur (20) an einem in Fließrichtung gesehen dem Stechelement (14) nachgeordneten und der Sammelstelle (18) vorgeordneten Abschnitt (32) des Kanals (16) auf dem Träger (12) angeordnet ist.

5. Probenentnahmesystem nach einem der Ansprüche 1 bis 4, **dadurch gekennzeichnet, dass** das Stechelement (14) als Spitze (22) an einer Schaftpartie (26) des Trägers (12) absteht, und dass die Aufnahmestruktur (20) im Bereich der Schaftpartie (26) angeordnet ist.

6. Probenentnahmesystem nach einem der Ansprüche 1 bis 5, **dadurch gekennzeichnet, dass** die Aufnahmestruktur (20) durch ein unter Freihaltung eines Kapillarspalts (30) gegenüber dem Kanal (16) an dem Träger (12) angeordnetes Deckelelement gebildet ist.

7. Probenentnahmesystem nach Anspruch 6, **dadurch gekennzeichnet, dass** der Kapillarspalt (30) eine geringere Kapillarattraktion für die Probenflüssigkeit als der daran angrenzende Kanalabschnitt (32) aufweist.

8. Probenentnahmesystem nach einem der Ansprüche 1 bis 7, **dadurch gekennzeichnet, dass** die Aufnahmestruktur (20) durch mindestens einen seitlich neben dem Kanal (16) auf dem Trägerteil angeordnete, vorzugsweise halboffene Überlaufkapillare (38) gebildet ist.

9. Probenentnahmesystem nach einem der Ansprüche 1 bis 8, **dadurch gekennzeichnet, dass** zwei Überlaufkapillaren (38) beidseitig von dem Kanal (16) vorzugsweise symmetrisch zueinander angeordnet sind.

10. Probenentnahmesystem nach Anspruch 8 oder 9, **dadurch gekennzeichnet, dass** die mindestens eine Überlaufkapillare (38) mindestens eine Verzweigung (40) aufweist.

11. Probenentnahmesystem nach einem der Ansprüche 8 bis 10, **dadurch gekennzeichnet, dass** der Kanal (16) durch eine Seitenwandung (42) von der mindestens einen Überlaufkapillare (38) getrennt ist, wobei überschüssige Probenflüssigkeit über die Seitenwandung (42) in die Überlaufkapillare (38) überfließt.

12. Probenentnahmesystem nach einem der Ansprüche 8 bis 10, **dadurch gekennzeichnet, dass** der Kanal (16) über eine kapillare Abzweigung (46) mit der mindestens einen Überlaufkapillare (38) fluidisch verbunden ist.

13. Probenentnahmesystem nach Anspruch 12, **dadurch gekennzeichnet, dass** die Abzweigung (46) einen kleineren Fließquerschnitt als die anschließende Überlaufkapillare (38) aufweist.

14. Probenentnahmesystem nach einem der Ansprüche 1 bis 13, **dadurch gekennzeichnet, dass** die Aufnahmestruktur (20) zugleich ein Reservoir für die Rückfüllung des Kanals (16) bildet.

15. Probenentnahmesystem nach einem der Ansprüche 1 bis 14, **dadurch gekennzeichnet, dass** das Stechelement (14) an einem Flachformteil als Träger (12) angeformt ist, und dass der Kanal (16) rillenförmig linear ausgebildet ist.

16. Probenentnahmesystem nach einem der Ansprüche 1 bis 15, **dadurch gekennzeichnet, dass** der Träger (12) und das Stechelement (14) durch photochemisches Maskenätzen einstückig aus einem Flachmaterial gebildet sind.

17. Blutanalysegerät mit mindestens einem vorzugsweise als Einwegprodukt ausgebildeten Probenentnahmesystem (10) nach einem der vorhergehenden Ansprüche.
